# DEMANDE DE BREVET EUROPEEN

(11) **EP 1 059 062 A1**
(43) Date de publication de la demande: **13.12.2000**
(21) Numéro de dépôt: 99870119.7
(22) Date de dépôt: 07.06.1999
(51) Int. Cl.: A61B 5/0205, A61B 5/113, A61B 5/08

(54) **Système de traitement de données destiné au diagnostic et/ou au suivi d'affections respiratoires**

(71) Demandeur: UNIVERSITE CATHOLIQUE DE LOUVAIN, B-1348 Louvain-la-Neuve (BE); Cliniques Saint-Luc A.S.B.L., 1200 Bruxelles (BE)
(72) Inventeur: Thys, Frédéric, 1300 Wavre (BE); Roeseler, Jean, 1150 Bruxelles (BE); Reynaert, Marc, 3080 Tervuren (BE)
(74) Mandataire: Van Malderen, Joelle

(57) **Abrégé**

La présente invention concerne un système de traitement de données physiologiques permettant de collecter et d'enregistrer sur une même échelle de temps plus de trois données physiologiques provenant d'un patient susceptible de souffrir d'affections respiratoires.

## Description

### Objet de l'invention

La présente invention est relative à un nouveau système de traitement de données et à un système incorporant ce système destiné au diagnostic et/ou au suivi d'affections respiratoires.

### Arrière-plan technologique et état de la technique

Dans les hôpitaux, en particulier dans les services d'urgences, le caractère imprévisible des accidents survenant aux patients ainsi que le manque d'espace vital, le temps de passage réduit et surtout le caractère urgent du diagnostic et de l'intervention thérapeutique, nécessitent de disposer d'instruments permettant d'améliorer ce diagnostic et d'assurer un suivi thérapeutique le moins traumatisant possible pour le patient.

Chez les patients admis dans un service d'urgence pour insuffisance respiratoire aiguë, l'altération de l'état de conscience, la présence d'arythmies sévères, l'épuisement musculaire et le développement d'une acidose respiratoire, malgré un traitement médical, nécessitent le plus souvent le recours à une ventilation mécanique. Celle-ci aura comme buts principaux la correction de l'hypoxémie artérielle et de l'acidose respiratoire aiguë, la récupération et la diminution de la consommation en oxygène des muscles respiratoires, la suppression de la dyspnée et le retour à l'état clinique existant avant l'événement aigu déstabilisant. Depuis plusieurs années, les cliniciens confrontés à l'insuffisance respiratoire aiguë essaient d'éviter l'intubation endotrachéale (I.E.T.) et la ventilation mécanique afin de diminuer les complications liées d'une part à l'intubation endotrachéale (traumatisme du pharynx, du larynx et de la trachée, hémorragie endotrachéale, complications infectieuses), et d'autre part à la ventilation mécanique (barotraumatisme, volutraumatisme) et leurs conséquences (pneumonie nosocomiale, nécessité d'analgésie et de sédation, augmentation de la durée de séjour, impossibilité de communication, etc.).

Lors du traitement postopératoire immédiat des sujets sans pathologie respiratoire préexistante ainsi que dans les services de soins intensifs de pneumologie, la ventilation non invasive, soit avec respirateur volumétrique, soit avec respirateur barométrique à deux niveaux de pression, via un masque facial, a démontré son efficacité pour éviter l'intubation endotrachéale et la ventilation mécanique dans une variété de situations cliniques (1,2,3,4,5). Actuellement, son utilité est bien reconnue dans la prise en charge de l'oedème pulmonaire aigu et de la décompensation aiguë d'une broncho-pneumopathie obstructive chronique (BPCO) (6,7). Celle-ci commence à être étudiée dans la prise en charge de l'asthme sévère (8). Par ailleurs, l'utilisation de cette méthode alternative dans un service d'urgence pour la prise en charge de la détresse respiratoire aiguë tend à s'imposer (9) .

A ce jour, différents dispositifs ou procédés ont été proposés pour analyser de manière indépendante certaines données relatives aux caractéristiques physiologiques du patient, tels que des dispositifs permettant une mesure et un enregistrement de la fréquence cardiaque et respiratoire, l'utilisation d'électromyographes permettant la mesure et l'enregistrement de contractions musculaires, des dispositifs de mesure et d'enregistrement de la pression oesophagienne, etc.

Cependant, aucun système de traitement de données adéquat n'a été proposé pour améliorer un diagnostic rapide et assurer le suivi des différents médicaments ou procédés thérapeutiques utilisés dans le cas de telles insuffisances respiratoires, en particulier dans les situations d'urgence.

### Buts de l'invention

La présente invention vise à fournir un système de traitement de données destiné à améliorer le diagnostic et/ou le suivi (monitoring) d'un patient, en particulier un patient souffrant d'affections respiratoires, et qui permet l'acquisition, l'enregistrement et éventuellement la lecture simultanés de données physiologiques d'un patient.

Un autre but de la présente invention vise à fournir un tel système qui soit d'une conception simple, incorporé dans un dispositif d'un encombrement minimal, c'est-à-dire pouvant par exemple être utilisé entre deux brancards, de préférence mobile et de conception ergonomique, tout en assurant une totale sécurité du patient et la conservation des données enregistrées et analysées.

### Eléments caractéristiques de l'invention

La présente invention est relative à un système de traitement de données permettant de traiter, c'est-à-dire de collecter et d'enregistrer sur une même échelle de temps plus de trois données physiologiques provenant d'un patient susceptible de souffrir d'affections respiratoires.

On entend par "données physiologiques issues d'un patient souffrant d'affections respiratoires", une mesure en fonction du temps ou "signal" de la fréquence cardiaque, de la fréquence respiratoire, de la saturation en oxygène et de la pression artérielle, des données résultant d'un électrocardiogramme, la variation en fonction du temps du volume thoracique abdominal et total du patient ainsi que des données physiologiques résultant des variations musculaires (signaux EMG), en particulier des muscles sterno-cléido-mastoïdiens et des trapèzes, ainsi que la variation de en fonction du temps de la pression (EMG) diaphragmatique.

De manière avantageuse, le système de traitement des données permet également de traiter d'autres signaux ou données provenant d'un électroencéphalogramme ou des signaux ou données provenant d'un module d'assistance respiratoire invasive ou de préférence non invasive placé sur le patient, en particulier les variations en fonction du temps de la pression inspiratoire et expiratoire délivrée, les variations en fonction du temps du débit et du volume insufflé, l'identification de fuites éventuelles, etc.

Le système de traitement de données de l'invention est avantageusement compris dans un dispositif destiné au diagnostic et au suivi des affections, en particulier des affections respiratoires, d'un patient, qui comprend, outre le système de traitement des données de l'invention, des éléments pour assurer l'acquisition, l'enregistrement et éventuellement la lecture simultanés des données physiologiques collectées. Ces moyens d'acquisition comportent différents senseurs disposés sur le patient et connectés au système de traitement des données, un ou plusieurs moyens d'enregistrement des différentes données (de manière simultanée ou séquentielle) constitués par exemple par un ordinateur, de préférence de type PC, incorporant éventuellement un graveur de CD-ROM, et un moyen de lecture constitué par un écran visualisant de manière simultanée les différentes données collectées qui apparaissent selon la même échelle de temps. Ces différentes données collectées peuvent également apparaître séparément ou groupées en un ensemble de données choisies pour une analyse plus précise par le personnel hospitalier, par exemple via une manipulation du clavier de commande de l'ordinateur.

Un autre aspect de la présente invention concerne un procédé de diagnostic et/ou de suivi d'affections, en particulier d'affections respiratoires, affectant un patient, dans lequel :
- on fixe différents senseurs et éventuellement un module d'assistance respiratoire sur le patient,
- on collecte par le système de traitement de données de l'invention de manière instantanée et simultanée différentes données physiologiques issues des senseurs,
- on établit une corrélation entre l'évolution de ces différentes données physiologiques et un diagnostic et/ou suivi d'une éventuelle insuffisance respiratoire affectant le patient.

Dans le présent procédé, aucun diagnostic direct sur le corps humain ou animal n'est effectué, mais ledit diagnostic est interprété par une personne compétente dans le domaine technique et médical à partir des données variables collectées, traitées, visualisées et enregistrées par le dispositif de l'invention.

Il est bien entendu que le système de traitement de données, le dispositif et le procédé de diagnostic et/ou de suivi de l'invention peuvent être adaptés par l'homme du métier en fonction de différentes variantes d'exécution de manière à améliorer son efficacité, réduire son encombrement et augmenter son ergonomie.

L'objet de l'invention sera décrit de manière détaillée dans l'exemple d'exécution ci-dessous en référence aux figures annexées.

### Brève description des figures

La figure 1 représente de manière schématique le dispositif de l'invention.

La figure 2 représente une vue partielle du dispositif de la figure 1 ainsi que les différentes connexions des senseurs transmettant les données physiologiques enregistrées sur le patient vers le système de traitement des données de l'invention.

### Description détaillée de l'invention

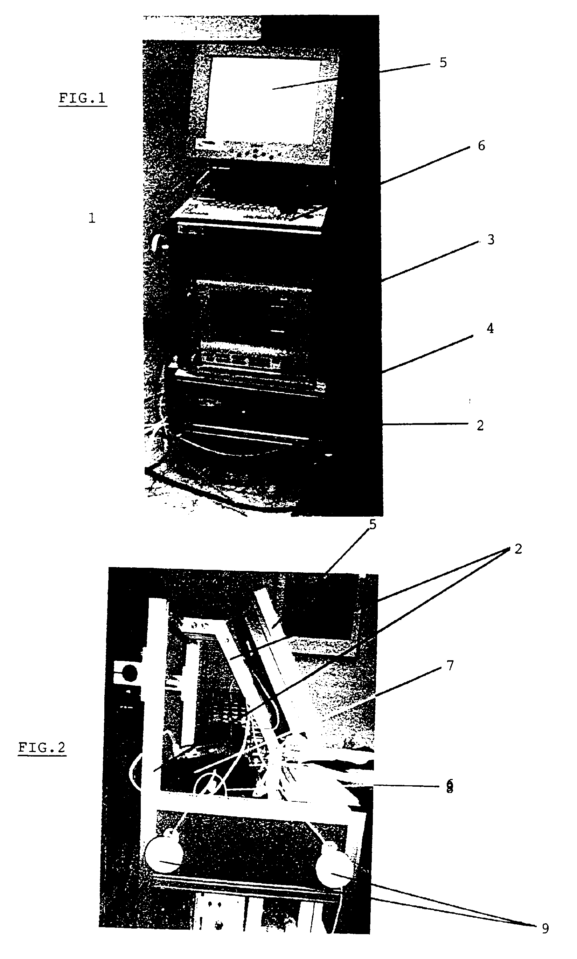

Le dispositif de l'invention, dénommé ci-après "module de monitoring" 1 et tel que représenté de manière schématique à la figure 1, comprend, posé sur un chariot 2 en aluminium, fermé et mobile sur roulettes (hauteur 112 cm, largeur 42 cm, profondeur 42 cm), un ordinateur 3, de préférence de type PC, muni d'une carte A/D 32 canaux avec filtre de garde (fréquences d'échantillonnage variables : 200 Hz pour ECG, 100 Hz pour EMG, 25 Hz pour les signaux respiratoires, 1 Hz pour les signaux lents (SPO2)) incorporant le système de traitement des données de l'invention. L'ordinateur est également relié à un dispositif d'enregistrement de certaines données que l'on souhaite conserver de manière permanente (par exemple un graveur de CD-ROM 4) à un écran 5 plat posé sur une tablette et visualisant de manière simultanée sur la même échelle de temps les différentes données physiologiques collectées sur le patient.

Les différents signaux ou données apparaissant sur cet écran de lecture 5 peuvent être avantageusement retraités par l'ordinateur 3 de manière automatique ou manuelle via un clavier de commande 6.

Le système de traitement des données de l'invention reçoit les différents signaux traités de senseurs 7 disposés sur le patient par des connexions 8 donc certaines sont représentées à la figure 2, en particulier une fiche de connexion de type DIN permettant d'enregistrer directement les données provenant d'un respirateur de type non invasif disposé sur la face du patient. Les différentes connexions peuvent être avantageusement disposées sur des systèmes d'enroulement passif de câbles 9 fixés sur les montants du chariot.

Les autres données sont des signaux résultant d'un suivi (monitoring) classique du patient au moyen de senseurs 7 disposés de préférence de manière non invasive directement sur le patient. Les données physiologiques captées par ces senseurs sont notamment une mesure de la fréquence cardiaque, de la fréquence respiratoire, de la saturation en oxygène (SpO2) obtenue par une oxymétrie du pouls avec une pince sonde apposée à l'extrémité d'un doigt (un canal pour l'enregistrement de données), de l'EtCO2 (capnographie) obtenue par l'introduction d'un cathéter dans le circuit respiratoire raccordé à un analyseur de CO₂ (module DATEX®) (signaux enregistrés sur un canal), du signal ECG obtenu par trois électrodes de surface collées sur le thorax (signaux enregistrés sur un canal), de la mesure artérielle (invasive ou non invasive) (signaux enregistrés sur un canal).

L'ensemble de ces signaux analogiques sont collectés par un moniteur DATEX® 9 (Helsinki, Finlande), de préférence un modèle compact AS3 DATEX/Engstrom. Les signaux disponibles sur l'écran de ce moniteur avec ses alarmes propres sont ensuite transmis par câble au module de monitoring de l'invention qui centralise et analyse tous les signaux.

Les signaux ou données collectés peuvent également provenir d'un senseur permettant l'enregistrement du volume thoracique, abdominal et total. Ces signaux sont obtenus de manière avantageuse par une pléthysmographie respiratoire à inductance. Ce système est constitué de deux bandes "ressort" incorporées dans un Téflon isolé (module Respibands, Respitrace®, Ambulatory Monitoring, Ardsley, New York, USA) placées autour du thorax et de l'abdomen. Les changements de volume thoracique ou abdominal produisent des changements dans l'inductance propre des bandes, et sont ensuite transmis via un câble transducteur au pléthysmographe (Respitrace®, Ambulatory Monitoring, Ardsley, New York, USA). Ces signaux (3 canaux) sont transmis via un câble au module de monitoring de l'invention.

Le système de traitement des données peut également traiter les données ou signaux provenant d'un électromyographe permettant la mesure et l'enregistrement de signaux EMG des muscles sterno-cléido-mastoïdiens et des trapèzes. Ces signaux sont obtenus par des électrodes autocollantes de surface (électrodes prégélifiées Ag/AgCi, Réf. P32MO (Numéris)) placées au niveau des muscles, une électrode de référence étant placée au niveau de la région mastoïdienne. Ces signaux (4 canaux, 8 électrodes et une électrode de référence) sont connectés à un système d'amplification tel que le système MEDATEC/Morpheus, Bruxelles, Belgique), fourni avec une alimentation séparée IEC 6(1-1). Les signaux analogiques issus du système d'amplification sont transmis via un câble au module de monitoring de l'invention.

Les signaux ou données peuvent être également issus d'une mesure de pression EMG diaphragmatique, le signal étant obtenu par une sonde oesophagienne souple bipolaire (Solid-state, Gaeltec CTO-2) introduite via une narine. Les signaux sont amplifiés par un amplificateur (2 canaux) et sont transmis via un câble au module de monitoring de l'invention. Cette mesure permet de préférence l'enregistrement de deux niveaux de pression sus- et sous-diaphragmatique.

Les signaux ou données peuvent également provenir d'un module d'assistance respiratoire non invasive. Ces signaux comprennent les mesures des pressions utilisées, du volume fourni par le respirateur, du débit de l'appareil, des fuites éventuelles et de la FiO2. Le respirateur utilisé est de préférence du type BIPAP S/T-D Hospital System avec système de monitoring propre BIPAP S/T-D 30 Detachable Control Panel (DCP) distribué par Respironics Inc.®, Pennsylvanie, USA). Les signaux du respirateur (courbes des pressions inspiratoires et expiratoires délivrées, courbe de débit et volume, fuites éventuelles) mesurées par le DCT sont obtenus par des sorties analogiques (DC) et transmises via un câble DIN au module de monitoring de l'invention.

Les signaux résultant des différentes données physiologiques collectées sont traitées par un système informatique adapté pour traiter plus de trois signaux ou données physiologiques sur une même échelle de temps. Le logiciel est adapté pour permettre une acquisition totale et de qualité des différentes données, pour assurer la transformation éventuelle desdites données, permettre une sélection de zones spécifiques (lecture appropriée de l'amplitude et de la durée d'un signal collecté et enregistré). En outre, ce logiciel assure l'enregistrement et éventuellement la conservation des différentes données collectées et est de préférence corrélé à une identification et à un classement des patients investigués de manière à faciliter le diagnostic et le suivi des différentes pathologies pouvant affecter un patient ou un groupe de patients.

En outre, le logiciel assure la conservation des différents signaux en rapport avec les données physiologiques collectées soit sur le disque dur de l'ordinateur, soit sur un autre support tel qu'un CD-ROM, ou l'impression des données sur un support papier. Ce logiciel a été adapté à partir d'un logiciel de polysomnographie (logiciel Morphée commercialisé par la société Medatec, Bruxelles, Belgique).

### REFERENCES

1. Meyer T.J. & Hill N.S., *Ann. Intern. Med.* **120**, pp. 760-770 (1994)
2. Freichels T.A., *Am. J. Crit. Care* **3**, pp. 6-10 (1994)
3. Pennock B.E., *Chest* **105**, pp. 441-444 (1994)
4. Wysocki M. et al., *Chest* **103**, pp. 907-913 (1993)
5. Foglio C. et al., *Chest* **101**, pp. 1533-1538 (1992)
6. Meduri G.U., *Clin. Chest. Med.* **17**, pp. 513-553 (1996)
7. Meduri G.U. et al., *Chest* **100**, pp. 445-454 (1991)
8. Meduri G.U. et al., *Chest* **110(3)**, pp. 767-774 (1996)
9. Pollack C.V. et al., *Ann. Emerg. Med.* **27**, pp. 189-192 (1996)

## Revendications

1. Système de traitement de données physiologiques permettant de collecter et d'enregistrer sur une même échelle de temps plus de trois données physiologiques provenant d'un patient susceptible de souffrir d'affections respiratoires.

2. Système selon la revendication 1, caractérisé en ce que les données physiologiques sont une mesure en fonction du temps de la fréquence cardiaque, de la fréquence respiratoire, de la saturation en oxygène et de la pression artérielle, des données résultant d'un électrocardiogramme, de la variation en fonction du temps du volume thoracique, abdominal et total d'un patient, de la variation musculaire en fonction du temps, en particulier de la variation en fonction du temps des muscles sterno-cléido-mastoïdiens et des trapèzes, et de la variation en fonction du temps de la pression diaphragmatique dudit patient.

3. Système selon la revendication 2, caractérisé en ce qu'il permet également de collecter et d'enregistrer sur une même échelle de temps que lesdites données physiologiques, des données concernant la variation de la pression inspiratoire et expiratoire délivrée et la variation du débit et du volume d'un module d'assistance respiratoire invasif ou non invasif placé sur le patient.

4. Dispositif de diagnostic et de suivi d'affections, en particulier d'affections respiratoires, d'un patient, comprenant le système de traitement de données selon l'une quelconque des revendications précédentes et des éléments pour assurer l'acquisition et l'enregistrement et éventuellement la lecture simultanée des donnes physiologiques dudit système de traitement des données.

5. Dispositif selon la revendication 4, caractérisé en ce que les éléments d'acquisition, d'enregistrement et de lecture comportent un ou plusieurs senseurs 7 disposés sur le patient et connectés au système de traitement des données, un ordinateur 3, incorporant éventuellement un graveur de CD-ROM 4, et un écran de lecture 5 permettant de visualiser de manière instantanée les différentes données collectées.

6. Dispositif selon la revendication 4 ou 5, caractérisé en ce que les différents éléments d'acquisition, d'enregistrement et de lecture sont posés sur un chariot mobile 2.

7. Procédé de diagnostic et/ou de suivi d'affections, en particulier d'affections respiratoires, affectant un patient, dans lequel :
- on fixe sur le patient un ou plusieurs senseurs 7 et éventuellement un module d'assistance respiratoire,
- on collecte par le système de traitement de données selon l'une quelconque des revendications 1 à 3, de manière instantanée et simultanée, différentes données physiologiques issues du ou des senseurs 7 sur une même échelle de temps,
- on établit par le dispositif selon l'une quelconque des revendications 4 à 6 une corrélation entre l'évolution de ces différentes données physiologiques et un diagnostic et/ou un suivi d'une éventuelle insuffisance respiratoire affectant ledit patient.
